(19) Europäisches Patentamt
European Patent Office
Office européen des brevets

(11) Veröffentlichungsnummer: **0 388 739 A1**

(12) # EUROPÄISCHE PATENTANMELDUNG

(21) Anmeldenummer: **90104562.5**

(22) Anmeldetag: **09.03.90**

(51) Int. Cl.5: **C07C 219/00, C07C 323/22, C07D 307/46, A61K 31/215**

(30) Priorität: **20.03.89 CH 1074/89**

(43) Veröffentlichungstag der Anmeldung:
**26.09.90 Patentblatt 90/39**

(84) Benannte Vertragsstaaten:
**AT BE CH DE DK ES FR GB GR IT LI LU NL SE**

(71) Anmelder: **F. HOFFMANN-LA ROCHE & CO. Aktiengesellschaft**
**Postfach 3255**
**CH-4002 Basel(CH)**

(72) Erfinder: **Hengartner, Urs, Dr.**
**Binzenstrasse 28**
**CH-4058 Basel(CH)**
Erfinder: **Ramuz, Henri, Prof., Dr.**
**Rheinparkstrasse 3**
**CH-4127 Birsfelden(CH)**

(74) Vertreter: **Lederer, Franz, Dr. et al**
**Patentanwalt Dr. Franz Lederer**
**Lucile-Grahn-Strasse 22**
**D-8000 München 80(DE)**

(54) **Tetrahydronaphtalinderivate.**

(57) Es wurde festgestellt, dass die neuen Tetrahydronaphthalinderivate der Formel

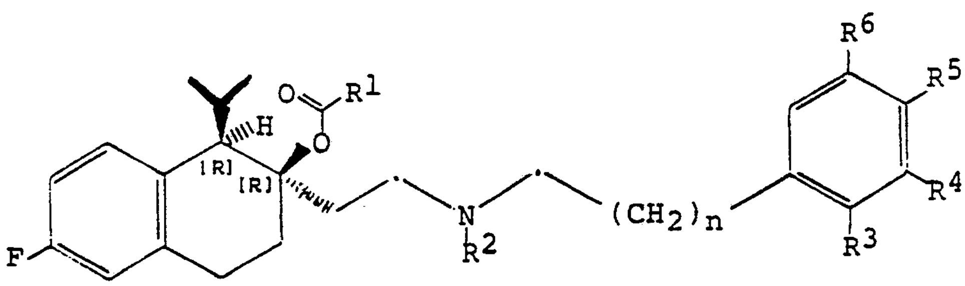

I

worin $R^1$ bis $R^6$ und n die in Anspruch 1 angegebene Bedeutung besitzen,
eine ausgeprägte der cerebralen Insuffizienz entgegenwirkende bzw. die cognitiven Funktionen verbessernde Wirkung aufweisen und auch die Mehrfach-Resistenz gegenüber Cytostatika bei der Behandlung von Tumoren bzw. Chloroquinresistenz bei der Behandlung von Malaria vermindern zu mögen. Sie können demnach als Arzneimittel verwendet werden, insbesondere für die Bekämpfung bzw. Verhütung der cerebralen Insuffizienz bzw. für die Verbesserung cognitiver Funktionen. Die Verbindungen der Formel I können durch O-Acylierung einer ebenfalls neuen Verbindung der Formel

EP 0 388 739 A1

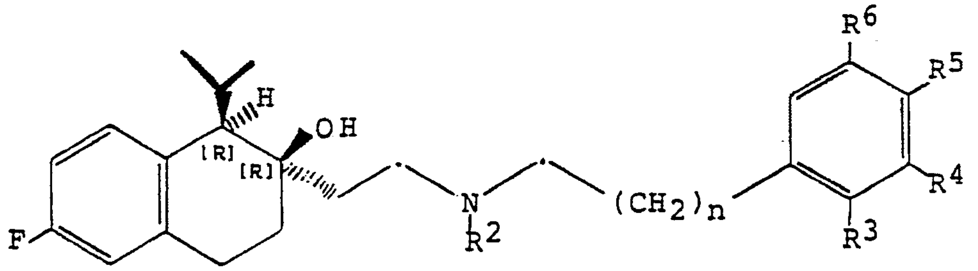

II

hergestellt werden.

## Tetrahydronaphthalinderivate

Die vorliegende Erfindung betrifft Tetrahydronaphthalinderivate. Im speziellen betrifft sie Tetrahydronaphthalinderivate der allgemeinen Formel

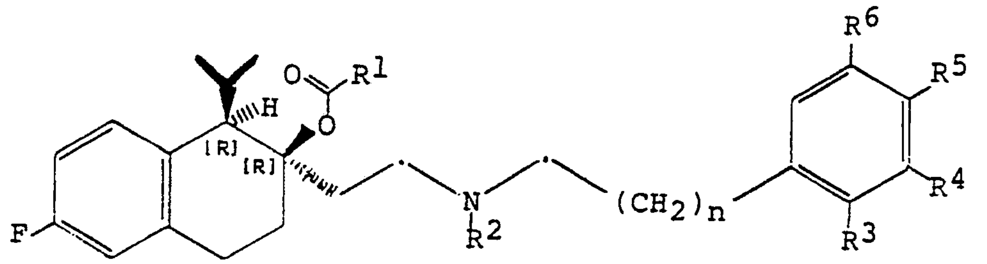

I

worin $R^1$ nieder-Alkyl, nieder-Alkoxy-nieder-alkyl, nieder-Alkylthio-nieder-alkyl oder Furyl, $R^2$ nieder-Alkyl, Phenyl-nieder-alkyl oder Cyclohexyl-nieder-alkyl, $R^3$ und $R^6$ je Wasserstoff oder Fluor, $R^4$ und $R^5$ je Wasserstoff oder nieder-Alkoxy oder zusammen Methylendioxy, Aethylendioxy oder Aethylenoxy und n die Zahl 1, 2 oder 3 bedeuten,
sowie pharmazeutisch verwendbare Säureadditionssalze davon.

Diese Verbindungen sind neu und zeichnen sich durch wertvolle pharmakodynamische Eigenschaften aus.

Gegenstand der vorliegenden Erfindung sind Verbindungen der allgemeinen Formel I und)- pharmazeutisch verwendbare Säureadditionssalze davon als solche und zur Anwendung als therapeutische Wirkstoffe, Verfahren zur Herstellung dieser Verbindungen, ferner diese enthaltende Arzneimittel und die Herstellung solcher Arzneimittel, sowie die Verwendung von Verbindungen der allgemeinen Formel I und von pharmazeutisch verwendbaren Säureadditionssalzen davon bei der Bekämpfung bzw. Verhütung von Krankheiten bzw. bei der Verbesserung der Gesundheit, insbesondere bei der Bekämpfung bzw. Verhütung der cerebralen Insuffizienz bzw. bei der Verbesserung cognitiver Funktionen, die Verwendung von Verbindungen der allgemeinen Formel I und von pharmazeutisch verwendbaren Säureadditionssalzen davon zur Verminderung der Mehrfach-Resistenz gegenüber Cytostatika bei der Behandlung von Tumoren bzw. Chloroquinresistenz bei der Behandlung von Malaria und die Verwendung von Verbindungen der allgemeinen Formel I und von pharmazeutisch verwendbaren Säureadditionssalzen davon zur Herstellung von Arzneimitteln für die Bekämpfung bzw. Verhütung der cerebralen Insuffizienz bzw. für die Verbesserung cognitiver Funktionen.

Der Formel I entsprechende [1S,2S]-Verbindungen. d.h. solche, die sich nur durch die relative Konfiguration in der 1- und 2-Stellung von den erfindungsgemässen Verbindungen unterscheiden, sind beispielsweise aus der US Patentschrift 4.680.310 bekannt. Diese besitzen eine ausgeprägte Calcium-antagonistische Wirkung und eignen sich zur Behandlung von Angina pectoris, Ischämie, Arrhythmien und Bluthochdruck.

Der in der vorliegenden Beschreibung verwendete Ausdruck "nieder-Alkyl" - allein oder in Kombination - bedeutet geradkettige und verzweigte, gesättigte Kohlenwasserstoffreste mit 1-4 Kohlenstoffatomen, d.h. Methyl, Aethyl, n-Propyl, Isopropyl, n-Butyl, Isobutyl, sec.-Butyl und tert.-Butyl. Der Ausdruck "nieder-Alkoxy" bedeutet nieder-Alkyläthergruppen, worin der Ausdruck "nieder-Alkyl" die obige Bedeutung hat. Der Ausdruck "Abgangsgruppe" bedeutet bekannte Gruppen wie Halogen, vorzugsweise Chlor oder Brom, Arylsulfonyloxy, wie etwa Tosyloxy, Brombenzolsulfonyloxy, Benzolsulfonyloxy oder Mesitylensulfonyloxy, oder Alkylsulfonyloxy, wie etwa Mesyloxy oder Trifluormethylsulfonyloxy.

Bevorzugt sind solche Verbindungen der Formel I, worin $R^1$ nieder-Alkoxy-nieder-alkyl oder Furyl, besonders bevorzugt Methoxymethyl oder 2-Furyl, bedeutet. n bedeutet vorzugsweise die Zahl 1. Weiter sind solche Verbindungen der Formel I bevorzugt, worin $R^2$ nieder-Alkyl, besonders bevorzugt Methyl, bedeutet. Ebenfalls bevorzugt sind die Verbindungen der Formel I, worin $R^3$ und $R^6$ je Wasserstoff und $R^4$ und $R^5$ je Wasserstoff oder nieder-Alkoxy, vorzugsweise Methoxy, oder zusammen Methylendioxy bedeuten.

Aus dem obigen folgt, dass solche Verbindungen der Formel I ganz besonders bevorzugt sind, worin $R^1$ Methoxymethyl oder 2-Furyl, $R^2$ Methyl, $R^3$ und $R^6$ je Wasserstoff, $R^4$ und $R^5$ je Wasserstoff, Methoxy oder zusammen Methylendioxy und n die Zahl 1 bedeuten.

Ganz speziell bevorzugte Verbindungen der Formel I sind:
[1R,2R]-(-)-6-Fluor-1,2,3,4-tetrahydro-1-isopropyl-2-[2-[methyl[3,4-(methylendioxy)phenäthyl]amino]äthyl]-2-naphthylmethoxyacetat,
[1R,2R]-(-)-2-[2-[(3,4-Dimethoxyphenäthyl)methylamino]äthyl]-6-fluor-1,2,3,4-tetrahydro-1-isopropyl-2-naphthylmethoxyacetat,
[1R,2R]-(-)-6-Fluor-1,2,3,4-tetrahydro-2-[2-[(p-methoxyphenäthyl)methylamino]äthyl]-1-isopropyl-2-naphthylmethoxyacetat,
[1R,2R]-(-)-6-Fluor-1,2,3,4-tetrahydro-1-isopropyl-2-[2-[methyl[3,4-(äthylendioxy)phenäthyl]amino]äthyl]-2-naphthylmethoxyacetat,
[1R,2R]-(-)-2-[2-[[3-(3,4-Dimethoxyphenyl)propyl]methylamino]äthyl]-6-fluor-1,2,3,4-tetrahydro-1-isopropyl-2-naphthylmethoxyacetat und
[1R,2R]-(-)-2-[2-[[4-(3,4-Dimethoxyphenyl)butyl]methylamino]äthyl]-6-fluor-1,2,3,4-tetrahydro-1-isopropyl-2-naphthylmethoxyacetat.

Die Verbindungen der Formel I sowie pharmazeutisch verwendbare Säureadditionssalze davon können hergestellt werden, indem man eine Verbindung der allgemeinen Formel

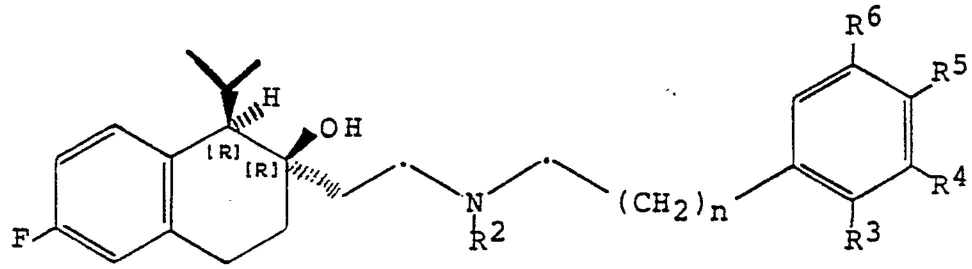

II

worin $R^2$, $R^3$, $R^4$, $R^5$, $R^6$ und n die oben angegebene Bedeutung besitzen,
mit einem eine nieder-Alkylcarbonyl-, nieder-Alkoxy-nieder-alkylcarbonyl-, nieder-Alkylthio-nieder-alkylcarbonyl- oder Furylcarbonyl-Gruppe abgebenden Acylierungsmittel umsetzt, und erwünschtenfalls, eine erhaltene Verbindung in ein pharmazeutisch verwendbares Säureadditionssalz überführt.

Die Acylierung einer Verbindung der Formel II erfolgt nach an sich bekannten Methoden. Geeignete Acylierungsmittel sind insbesondere aktivierte Säurederivate, wie Säurehalogenide und Säureanhydride oder gemischte Säureanhydride. Die Reaktion wird in einem unter den Reaktionsbedingungen inerten organischen Lösungsmittel oder Lösungsmittelgemisch bei einer Temperatur zwischen etwa 0° C und der Rückflusstemperatur durchgeführt. Als Lösungsmittel kommen insbesondere aromatische Kohlenwasserstoffe, wie Benzol, Toluol oder Xylol, chlorierte Kohlenwasserstoffe, wie Methylenchlorid oder Chloroform, Aether, (wie Diäthyläther, Tetrahydrofuran oder Dioxan, und dgl. in Frage.

Die Verbindungen der Formel II sind neu und ebenfalls Gegenstand der vorliegenden Erfindung. Sie können hergestellt werden, indem man eine Verbindung der allgemeinen Formel

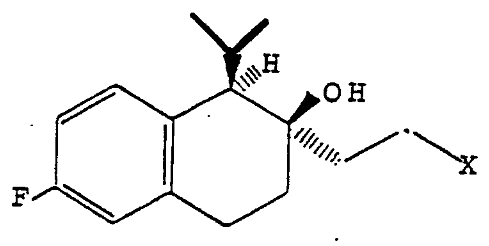

III

worin X eine Abgangsgruppe bedeutet,
mit einem Amin der allgemeinen Formel

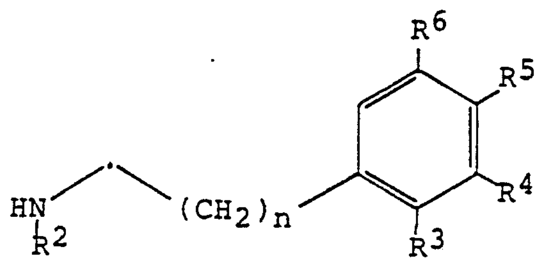

IV .

worin $R^2$, $R^3$, $R^4$, $R^5$, $R^6$ und n die oben angegebene Bedeutung besitzen, umsetzt.

Eine Verbindung der Formel III wird nach an sich bekannten Methoden mit einem Amin der Formel IV umgesetzt. Die Umsetzung erfolgt in Gegenwart oder Abwesenheit eines unter den Reaktionsbedingungen inerten organischen Lösungsmittels bei einer Temperatur zwischen etwa 20° und 150° C, vorzugsweise zwischen etwa 80° und 120° C. Bei dieser Umsetzung kommen Lösungsmittel, wie Dimethylformamid, Dimethylsulfoxid, Alkohole, wie Isopropanol oder tert.-Butanol, Aether, wie Tetrahydrofuran oder Dioxan, aromatische Kohlenwasserstoffe, wie Benzol, Toluol oder Xylol, chlorierte Kohlenwasserstoffe, wie Methylenchlorid, Tetrachlorkohlenstoff oder Chlorbenzol, und dgl. in Frage. Die Umsetzung erfolgt vorteilhafterweise in Gegenwart eines säurebindenden Mittels, wie eines tertiären Amins, wie Trimethylamin, Triäthylamin, Aethyldiisopropylamin oder 1,5-Diazabicyclo[4.3.0]non-5-en, wobei auch überschüssiges Amin der Formel IV als säurbindendes Mittel dienen kann. Aus Zweckmässigkeitsgründen arbeitet man bei Atmosphärendruck, obwohl höhere Drucke ebenfalls angewandt werden können.

Die Ausgangsstoffe der Formel III sind ebenfalls neu und Gegenstand der vorliegenden Erfindung. Ein Herstellungsverfahren ist im nachfolgenden Schema I skizziert. Bezüglich der genauen Reaktionsbedingungen wird auf den Beispielteil verwiesen.

Schema I

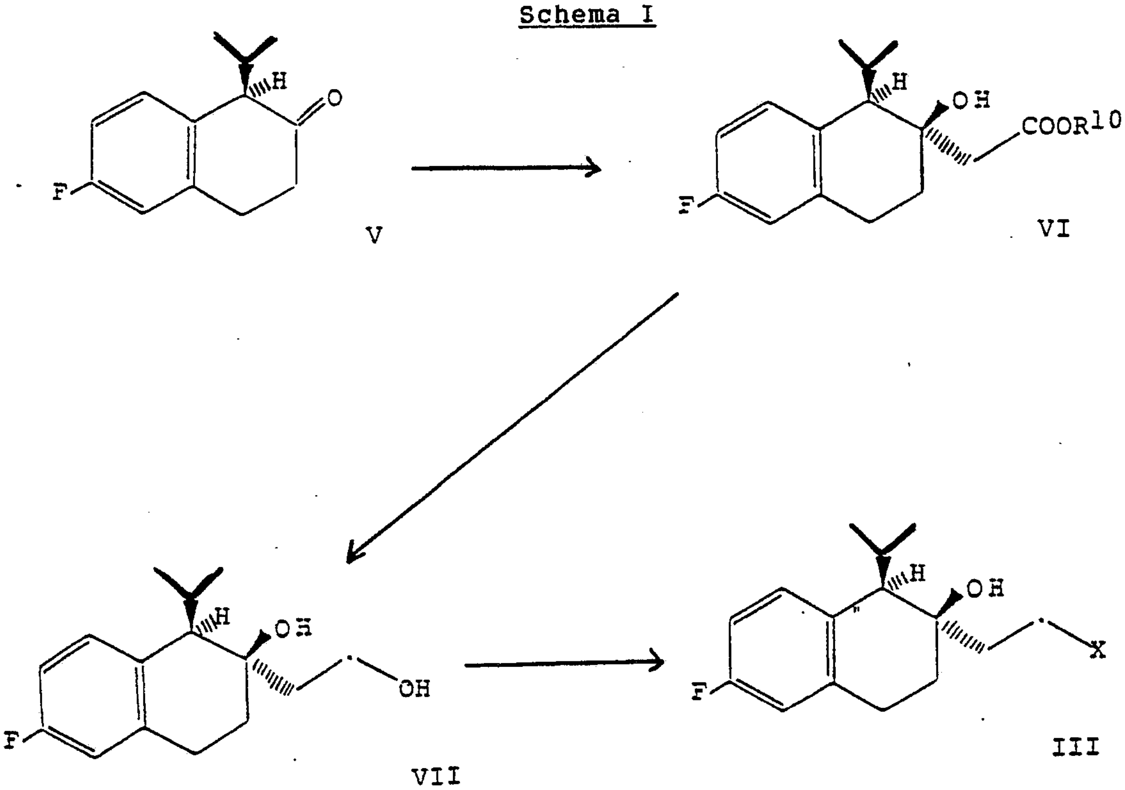

V

VI

VII

III

worin $R^{10}$ Wasserstoff oder nieder-Alkyl bedeutet.

In der ersten Stufe wird in an sich bekannter Weise ein Tetralonderivat der Formel V mit einem Halogenessisäurealkylester in Gegenwart von Zink umgesetzt, wobei die Umsetzung mit einem Halogenessigsäure-tert.-butylester auch in Gegenwart von Magnesium durchgeführt werden kann. Die Umsetzung erfolgt in einem inerten organischen Lösungsmittel oder Lösungsmittelgemisch, wie einem

Aether, wie Diäthyläther oder Tetrahydrofuran, oder einem aromatischen Kohlenwasserstoff, wie Benzol oder Toluol, oder Gemischen davon bei einer Temperatur zwischen etwa 0° C und der Rückflusstemperatur des Lösungsmittels. In situ-Zerlegung des intermediär gebildeten Additionsproduktes liefert einen Ester der Formel VI, welcher in an sich bekannter Weise zur entsprechenden Säure der Formel VI hydrolysiert werden kann.

Ein Ester oder eine Säure der Formel VI kann ebenfalls nach bekannten Methoden zum entsprechenden Alkohol der Formel VII reduziert werden. Geeignete Reduktionsmittel sind beispielsweise Lithiumaluminiumhydrid, Natrium-bis-(2-methoxyäthoxy)aluminiumhydrid, Lithiumborhydrid, Diisobutylaluminiumhydrid oder Diboran und dgl. Die Reduktion wird in einem unter den Reaktionsbedingungen inerten organischen, aprotischen Lösungsmittel, wie einem Aether, wie Diäthyläther, Tetrahydrofuran oder Dioxan, einem Kohlenwasserstoff, wie Hexan oder Cyclohexan, oder einem aromatischen Kohlenwasserstoff, wie Benzol oder Toluol, und dgl. bei einer Temperatur zwischen etwa Raumtemperatur und 100° C, vorzugsweise zwischen etwa Raumtemperatur und 50° C, durchgeführt.

Ein Alkohol der Formel VII kann durch Umsetzen mit einem Aryl- oder Alkylsulfonylhalogenid in bekannter Weise in eine Verbindung der Formel III überführt werden, worin X eine Aryl- oder Alkylsulfonylgruppe bedeutet. Diese Umsetzung erfolgt vorteilhafterweise in Gegenwart eines säurebindenden Mittels, wie eines tertiären Amins, wie Triäthylamin, Aethyldiisopropylamin oder Pyridin, in Gegenwart oder Abwesenheit eines unter den Reaktionsbedingungen inerten organischen Lösungsmittels bei einer Temperatur zwischen etwa 0° und 80° C. Als Lösungsmittel kommen Aether, wie Diäthyläther, Tetrahydrofuran oder Dioxan, aromatische, Kohlenwasserstoffe, wie Benzol oder Toluol, chlorierte Kohlenwasserstoffe, wie Methylenchlorid oder Chloroform, und dgl., Gemische davon oder überschüssiges säurebindendes Mittel in Frage. Eine Verbindung der Formel III, worin X Halogen bedeutet, erhält man zweckmässig durch Umsetzen einer Verbindung der Formel III, worin X Aryl- oder Alkylsulfonyl bedeutet, mit einem Natriumhalogenid in Aceton oder einem Pyridinhydrohalogenid bei einer Temperatur zwischen etwa 0° und 100° C.

Die Verbindungen der Formeln VI und VII sind neu und ebenfalls Gegenstand der vorliegenden Erfindung, während die Tetralonderivte der Formel V entweder bekannt sind oder in Analogie zur Herstellung der bekannten Verbindungen erhalten werden können.

Die ebenfalls als Ausgangsstoffe eingesetzten Amine der Formel IV sind entweder bekannt oder können in Analogie zur Herstellung der bekannten Verbindungen erhalten werden.

Die Verbindungen der allgemeinen Formel I enthalten zwei asymmetrische Zentren und liegen in der durch die Formel I angegebenen absoluten [R,R]-Konfiguration vor.

Wie eingangs erwähnt, sind die Tetrahydronaphthalinderivate der Formel I und deren pharmazeutisch verwendbare Säureadditionssalze neue Verbindungen mit äusserst wertvollen pharmakodynamischen Eigenschaften. Es hat sich gezeigt, dass sie der in dem nachstehend beschriebenen Tierversuch experimentell hervorgerufenen cerebralen Insuffizienz entgegenzuwirken vermögen.

Die Test-Apparatur ist eine "Skinner box" mit einem elektrifizierbaren Gitterboden (30 x 40 cm) und einer grauen Plastik-Plattform (15 x 15 x 0,8 cm) in der Ecke vorne rechts. Unerfahrene männliche Ratten (100-120 g) werden einzeln auf die Plattform gebracht. Sobald sie auf den Gitterboden hinabsteigen, erhalten sie einen elektrischen Fuss-Schock (0,8 mA). Die normale Reaktion unerfahrener Ratten ist, daraufhin auf die Plattform zurückzuspringen. Da jedoch die Ratten immer nochmals herabzuklettern versuchen, muss die Fuss-Schock-Prozedur für jedes Tier drei- bis fünfmal wiederholt werden. Nach diesen drei bis fünf Wiederholungen pro Tier haben die hatten eine sogenannte "passive avoidance response" erlernt, d.h. sie versuchen nicht mehr, auf den Gitterboden hinabzusteigen, weil sie wissen, dass sie bestraft werden.

Unmittelbar anschliessend werden drei Gruppen von je 30 Tieren gebildet. Die erste Gruppe erhält eine Injektion (i.p.) von 0,3 mg/kg Scopolamin sowie destilliertes Wasser (2 ml/kg p.o.). Die zweite Gruppe erhält eine Injektion (i.p.) von 0,3 mg/kg Scopolamin und eine orale Dosis der Testsubstanz. Die dritte Gruppe erhält ausschliesslich destilliertes Wasser (p.o.).

2 Stunden später wird jede Ratte einmal auf die Plattform in der "Skinner box" gesetzt. Das Kriterium für die Beurteilung dieses Tests zur Ermittlung einer Präparatwirkung auf das Kurzzeit-Gedächtnis ist, ob das Tier während 60 Sekunden auf der Plattform bleibt oder nicht (das Ergebnis kann also für jedes Tier nur "ja" oder "nein" lauten). Die statistische Signifikanz der Unterschiede zwischen den bei der ersten und der zweiten Gruppe erhaltenen Resultaten wird mittels des Chi-Quadrat-Tests ermittelt.

70 - 75% der lediglich mit destilliertem Wasser (p.o.) behandelten Tiere erinnern sich 2-4 Stunden nach Erlernen der "passive avoidance response" noch daran, dass sie auf der Plattform bleiben sollten. Bei 85-92% der mit Scopolamin (0,3 mg/kg i.p.) und destilliertem Wasser (p.o.) behandelten Tiere lässt sich während 3-4 Stunden ein retrograder Effekt auf das Kurzzeitgedächtnis feststellen, d.h. sie haben vergessen, dass sie auf der Plattform bleiben müssen. Eine Substanz, welche cerebraler Insuffizienz entgegenzuwirken vermag, kann die durch die Injektion (i.p.) von 0,3 mg/kg Scopolamin hervorgerufene Blockierung des Kurzzeitgedächtnisses aufheben. Eine Dosis eines Präparats wird dann als aktiv gegen Scopolamin bezeichnet, wenn die Anzahl der positiven Ergebnisse ("ja") von derjenigen mit Scopolamin (0,3 mg/kg i.p.) und nur destilliertem Wasser (p.o.) behandelter Kontroll-Tiere signifikant verschieden ist.

In der nachstehenden Tabelle ist angegeben, bei welchen Dosen bestimmte Verbindungen der Formel I in dem vorstehend beschriebenen Versuch eine signifikante Aktivität zeigen.

Tabelle

| $R^1$ | $R^2$ | $R^3$ | $R^4$ | $R^5$ | $R^6$ | n | Signifikant wirksame Dosen mg/kg p.o. |
|---|---|---|---|---|---|---|---|
| $CH_3OCH_2$ | $CH_3$ | H | $-O-CH_2-O-$ | | H | 1 | 0,001<br>0,003<br>0,01<br>0,03<br>0,1<br>0,3<br>1 |
| $CH_3OCH_2$ | $CH_3$ | H | $OCH_3$ | $OCH_3$ | H | 1 | 0,1<br>0,3<br>1<br>3 |
| $CH_3OCH_2$ | $CH_3$ | H | H | $OCH_3$ | H | 1 | 0,001<br>0,003<br>0,1<br>0,3<br>1<br>3<br>10<br>30 |
| $CH_3OCH_2$ | $CH_3$ | H | $-O-CH_2CH_2-O-$ | | H | 1 | 0,01<br>0,03<br>0,1<br>0,3 |
| $CH_3OCH_2$ | $CH_3$ | H | $OCH_3$ | $OCH_3$ | H | 2 | 0,3<br>1 |
| $CH_3OCH_2$ | $CH_3$ | H | $OCH_3$ | $OCH_3$ | H | 3 | 0,1<br>0,3 |

Die Verbindungen der Formel I und deren pharmazeutisch verwendbare Säureadditionssalze können als Heilmittel, z.B. in Form pharmazeutischer Präparate, Verwendung finden. Die pharmazeutischen Präparate können oral, z.B. in Form von Tabletten, Lacktabletten, Dragées, Hart- und Weichgelatinekapseln, Lösungen, Emulsionen oder Suspensionen, verabreicht werden. Die Verabreichung kann aber auch rektal, z.B. in Form von Suppositorien, oder parenteral, z.B. in Form von Injektionslösungen, erfolgen.

Wie weiter vorne erwähnt, sind Arzneimittel, enthaltend eine Verbindung der Formel I oder ein pharmazeutisch verwendbares Säureadditionssalz davon ebenfalls Gegenstand der vorliegenden Erfindung, weiterhin auch ein Verfahren zur Herstellung derartiger Arzneimittel, welches dadurch gekennzeichnet ist, dass man eine oder mehrere Verbindungen der Formel I und/oder pharmazeutisch verwendbare Säureadditionssalze davon und erwünschtenfalls einen oder mehrere andere therapeutisch wirksame Stoffe zusammen mit einem oder mehreren therapeutisch inerten, anorganischen oder organischen Exzipientien in eine galenische Darreichungsform bringt.

Zur Herstellung von Tabletten, Lacktabletten, Dragées und Hartgelatinekapseln kann man als Excipientien z.B. Lactose, Maisstärke oder Derivate davon, Talk, Stearinsäure oder deren Salze etc. verwenden.

Für Weichgelatinekapseln eignen sich als Excipientien z.B. vegetabile Oele, Wachse, Fette, halbfeste

und flüssige Polyole etc.

Zur Herstellung von Lösungen und Sirupen eignen sich als Excipientien z.B. Wasser, Polyole, Saccharose, Invertzucker, Glukose und dergleichen.

Für Injektionslösungen eignen sich als Excipientien z.B. Wasser, Alkohole, Polyole, Glyzerin, vegetabile Oele etc.

Für Suppositorien eignen sich als Excipientien z.B. natürliche oder gehärtete Oele, Wachse, Fette, halbflüssige oder flüssige Polyole und dergleichen.

Die pharmazeutischen Präparate können daneben noch Konservierungsmittel, Lösevermittler, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel oder Antioxidantien enthalten. Sie können auch noch andere therapeutisch wertvolle Stoffe enthalten.

Erfindungsgemäss kann man die Verbindungen der Formel I und pharmazeutisch verwendbare Säureadditionssalze davon zur Verminderung der Mehrfach-Resistenz gegenüber Cytostatika bzw. Chloroquinresistenz bei der Behandlung von Tumoren bzw. Malaria und insbesondere bei der Bekämpfung bzw. Verhütung der cerebralen Insuffizienz bzw. bei der Verbesserung cognitiver Funktionen (wie Erinnerungsvermögen, Lernfähigkeit, Interesse an der Umwelt und Selbstsorge) verwenden, beispielsweise in der Geriatrie, bei Intoxikationen, wie Alkoholismus, und bei cerebro-vaskulären Störungen; mögliche weitere Einsatzgebiete sind vestibuläre Störungen (wie Menière-Krankheit) und Entwicklungsstörungen (wie Dyslexie). Die Dosierung kann innerhalb weiter Grenzen variieren und ist natürlich in jedem einzelnen Fall den individuellen Gegebenheiten anzupassen. Im allgemeinen dürfte bei oraler Verabreichung eine Tagesdosis von etwa 25 bis 150 mg angemessen sein, wobei aber die soeben angegebene obere Grenze auch überschritten werden kann, wenn sich dies als angezeigt erweisen sollte.

Gegenstand der Erfindung ist schliesslich auch die Verwendung der Verbindungen der Formel I und von pharmazeutisch verwendbaren Säureadditionssalzen davon zur Herstellung von Arzneimitteln für die Bekämpfung bzw. Verhütung der cerebralen Insuffizienz bzw. für die Verbesserung cognitiver Funktionen.

Die folgenden Beispiele sollen die vorliegende Erfindung erläutern, sie jedoch in keiner Weise einschränken. Alle Temperaturen sind in Celcius Graden angegeben.

## Beispiel 1

Eine Lösung von 87,4 g (0,221 Mol) [1R,2R]-(-)-6-Fluor-1,2,3,4-tetrahydro-1-isopropyl -2-[2-[methyl[3,4-(methylendioxy)phenäthyl]amino]äthyl] -2-naphthalinol, 750 ml abs. Chloroform und 35,7 ml (0,21 Mol) Aethyldiisopropylamin wird unter Rühren bei -5° innerhalb 1 Stunde mit 45,6 g (38,6 ml, 0,42 Mol) Methoxyacetylchlorid tropfenweise versetzt und danach 16 Stunden ohne zusätzliche Abkühlung weitergerührt. Die Lösung wird mit Eiswasser versetzt, mit 350 ml 1N Natronlauge gewaschen, getrocknet und unter vermindertem Druck eingedampft. Das zurückbleibende Oel (115,6 g) wird in 50 ml Methanol gelöst und mit einer methanolischen Salzsäure-Lösung bis pH 2 versetzt. Zu dieser Lösung wird Aether bis zur schwachen Trübung gegeben. Nach 64-stündigem Stehenlasen im Kühlschrank werden die weissen Kristalle abgenutscht und aus Methanol und Aether umkristallisiert. So erhält man 84,6 g (77%) [1R,2R]-(-)-6-Fluor-1,2,3,4-tetrahydro-1-isopropyl -2-[2-[methyl[3,4-(methylendioxy)phenäthyl]amino]äthyl] -2-naphthylmethoxyacetat-hydrochlorid, Smp. 203-204°; $[\alpha]_D^{20}$ = -36,1° (c=1, Methanol).

Das als Ausgangsmaterial verwendete [1R,2R]-(-)-6-Fluor-1,2,3,4-tetrahydro-1-isopropyl-2-[2-[methyl-[3,4-(methylendioxy)phenäthyl]amino]äthyl]-2-naphthalinol wurde wie folgt hergestellt:

Zu einer Lösung von 204,1 g (1,04 Mol) rac. 2-(p-Fluorphenyl)-3-methylbuttersäure in 1200 ml Acetonitril wird unter Rühren eine Lösung von 63,0 g (0,52 Mol) [R]-(+)-1-Phenyläthylamin in 800 ml Acetonitril gegeben. Nach 1 Stunde wird der kristalline Niederschlag abfiltriert, mit 300 ml Acetonitril gewaschen und über Nacht unter vermindertem Druck bei 50° getrocknet). Nach wiederholtem Umkristallisieren aus Aethanol erhält man das entsprechende Salz als farblose Kristalle, Smp. 209-210°. Dieses Salz wird in Aether suspendiert und mit 3N Salzsäure bis pH 1 versetzt. Die ätherische Lösung wird mit Wasser gewaschen, getrocknet und unter vermindertem Druck eingedampft, wobei man [R]-(-)-2-(p-Fluorphenyl)-3-methylbuttersäure erhält, Smp. 56-57°; $[\alpha]_D^{20}$ = -48,4° (c=1, Methanol).

Eine Lösung aus 32.3 g (0,165 Mol) [R]-(-)-2-(p-Fluorphenyl)-3-methylbuttersäure in 250 ml abs. Methylenchlorid werden mit 27,3 ml Oxalylchlorid versetzt. Nach 4-stündigem Rühren bei Raumtemperatur und Eindampfen des Lösungsmittels unter vermindertem Druck erhält man [R]-(-)-2-(p-Fluorphenyl)-3-methylbuttersäurechlorid als Oel, Sdp. 100°/65 Pa, $[\alpha]_D^{20}$ = -53,2° (c=1, Chloroform).

Eine Lösung von 54,8 g (0,255 Mol) [R]-(-)-2-(p-Fluorphenyl)-3-methylbuttersäurechlorid in 950 ml

Methylenchlorid wird bei -10° mit Aethylengas gesättigt. Bei -56° werden danach 47,5 g (0,356 Mol) Aluminiumchlorid auf einmal zugegeben. Nach 15 Minuten gibt man nochmals 10,0 g (0,075 Mol) Aluminiumchlorid zu und lässt das Reaktionsgemisch langsam auf -20° erwärmen. Dann werden 500 ml Eiswaser vorsichtig zugegeben, und die organische Phase abgetrennt und mit 3N Salzsäur und Wasser gewaschen. Nach dem Trocknen und Eindampfen des Lösungsmittels unter vermindertem Druck erhält man 59,6 g [R]-(+)-6-Fluor-3,4-dihydro-1-isopropyl-2(1H)-naphton als ein leicht gelbes Oel; $[\alpha]_D^{20}$ = +189,5° (c=1, Methanol).

7,92 g (0,32 Grammatom) Magnesium werden mit 125 ml abs. Tetrahydrofuran bedeckt und 5 ml t-Butyl-bromacetat so zuge geben, dass die Reaktion startet. Danach wird innerhalb 30 Minuten eine Lösung von 62,2 g (0,319 Mol) t-Butyl-bromacetat und 52,6 g (0,255 Mol) [R]-(+)-6-Fluor-3,4-dihydro-1-isopropyl-2-(1H)-naphton in 375 ml abs, Tetrahydrofuran so zugegeben, dass das Reaktionsgemisch unter Rückfluss siedet. Nach beendeter Zugabe wird das Reaktionsgemisch zunächst 10 Minuten zum Rückfluss erhitzt, danach auf 15° abgekühlt und schliesslich mit einer Lösung von 40 g Ammoniumchlorid in 500 ml Wasser versetzt. Die organische Phase wird abgetrennt, mit Wasser gewaschen, über Natriumsulfat getrocknet und unter vermindertem Druck eingedampft, wobei man [1R,2R]-(-)-t-Butyl-6-fluor-1,2,3,4-tetrahydro-2-hydroxy -1-isopropyl-2-naphthylacetat als gelbes Oel erhält, welches ohne weitere Reinigung in die nächste Stufe eingesetzt wird.

Zu einer gekühlten Suspension von 19,4 g (0,51 Mol) Lithiumaluminiumhydrid in 500 ml abs. Tetrahydrofuran wird eine Lösung aus 82,2 g (0.255 Mol) [1R,2R]-(-)-t-Butyl-6-fluor-1,2,3,4-tetrahydro-2-hydroxy -1-isopropyl-2-naphthylacetat in 500 ml abs. Tetrahydrofuran innerhalb 30 Minuten so zugegeben, dass die Temperatur 30° nicht übersteigt. Nach weiterem Rühren während 30 Minuten wird das Reaktionsgemisch auf 5° abgekühlt und sorgfältig nacheinander mit ml Eiswasser. 30 ml 1N Natronlauge und 90 ml Eiswasser versetzt. Danach wird das Gemisch noch 60 Minuten gerührt und filtriert, und das Filtrat unter vermindertem Druck eingedampft. Der ölige Rückstand wird in einem 20:1-Gemisch von Chloroform und Methanol gelöst und an 900 g Kieselgel chromatographiert. wobei man [1R,2R]-(-)-2-[6-Fluor-1,2,3,4-tetrahydro-2-hydroxy -1-isopropyl-2-naphthyl]äthanol als weisse Kristalle erhält, Smp. 78-80°; $[\alpha]_D^{20}$ =83,1° (c=1, Methanol).

28,5 g (0,113 Mol) [1R,2R]-(-)-2-[6-Fluor-1,2,3,4-tetrahydro-2-hydroxy -1-isopropyl-2-naphthyl]äthanol werden in 150 ml Pyridin gelöst und bei 0° mit 31,5 g (0,165 Mol) p-Toluolsulfonylchlorid portionsweise innerhalb von 30 Minuten versetzt. Nach 90 Minuten wird die Reaktionslösung auf Eiswasser gegossen und mit Aether extrahiert. Die organische Phase wird nacheinander mit 3N Salzsäure, Wasser und einer gesättigten Natriumcarbonatlösung gewaschen. Nach dem Trocknen über Magnesiumsulfat und Eindampfen des Lösungsmittels unter vermindertem Druck verbleibt ein öliger Rückstand, welcher aus Aether/Hexan umkristallisiert wird, wobei man 38,8 g (85%) [1R,2R]-(-)-2-[6-Fluor-1,2,3,4-tetrahydro-2-hydroxy -1-isopropyl-2-naphthyl]äthyl-p-toluolsulfonat als weisse Kristalle erhält, Smp. 65-67°; $[\alpha]_D^{20}$ = -54,1° (c=1; Methanol).

Eine Lösung von 12,6 g (0,031 Mol) [1R,2R]-(-)-2-[-6-Fluor-1,2,3,4-tetrahydro-2-hydroxy -1-isopropyl-2-naphthyl]äthyl-p-toluolsulfonat, 6,1 g (0,031 Mol) 3,4-(Methylendioxy)-N-methyl-β-phenäthylamin und 5,3 ml (0,031 Mol) Aethyldiisopropylamin in 50 ml Dimethylsulfoxid wird unter einer Stickstoffatomosphäre 3 Stunden bei 80° gerührt und danach im Hochvakuum eingeengt. Der Rückstand wird in Methylenchlorid gelöst, die erhaltene Lösung mit einer gesättigten Natriumcarbonatlösung gewaschen und dreimal mit 3N Salzsäure extrahiert. Die wässrigen Extrakte werden mit einer 28%igen Natriumhydroxylösung alkalisch gestellt. Die ölige Suspension wird dreimal mit Methylenchlorid extrahiert, die organischen Extrakte getrocknet und unter vermindertem Druck eingedampft. Das zurückbleibende Oel wird an Kieselgel mit Essigester/Methanol (9:1) chromatographiert. So erhält man 9,3 g (73%) [1R,2R]-(-)-6-Fluor-1,2,3,4-tetrahydro-1-isopropyl -2-[2-[methyl[3,4-(methylendioxy)phenäthyl]amino]äthyl] -2-naphthalinol als ein gelbliches Oel, $[\alpha]_D^{20}$ = -22,1°.

In analoger Weise wie oben beschrieben wurden die folgenden Verbindungen hergestellt:

- Aus [1R,2R]-(-)-2-[6-Fluor-1,2,3,4-tetrahydro-2-hydroxy-1-isopropyl-2-naphthyl]äthyl-p-toluolsulfonat und N-Methylhomoveratrylamin das [1R,2R]-(-)-2-[2-[(3,4-Dimethoxyphenäthyl)methylamino]äthyl]-6-fluor-1,2,3,4-tetrahydro-1-isopropyl-2-naphthalinol-hydrochlorid, Smp. 165-166° (aus Essigester/Aether); $[\alpha]_D^{20}$ = -45,1° (c=1, Methanol);
- aus [1R,2R]-(-)-2-[6-Fluor-1,2,3,4-tetrahydro-2-hydroxy-1-isopropyl-2-naphthyl]äthyl-p-toluolsulfonat und N-Methyl-β-phenyläthylamin das [1R,2R]-(-)-6-Fluor-1,2,3,4-tetrahydro-1-isopropyl-2-[2-[(phenäthyl)-methylamino]äthyl]-2-naphthalinol als ein leicht gelbes Oel, welches direkt in die nächste Stufe eingesetzt wird;
- aus [1R,2R]-(-)-2-[6-Fluor-1,2,3,4-tetrahydro-2-hydroxy-1-isopropyl-2-naphthyl]äthyl-p-toluolsulfonat und N-Methyl-β-(p-methoxyphenäthylamin) das [1R,2R]-(-)-6-Fluor-1,2,3,4-tetrahydro-1-isopropyl-2-[2-[(p-methoxyphenäthyl)methylamino]äthyl]-2-naphthalinol als ein leicht gelbliches Oel, welches direkt in die nächste

Stufe eingesetzt wird;

- aus [1R,2R]-(-)-2-[6-Fluor-1,2,3,4-tetrahydro-2-hydroxy-1-isopropyl-2-naphthyl]äthyl-p-toluolsulfonat und 3,4-(Methylendioxy)-N-äthyl-β-phenyläthylamin das [1R,2R]-(-)-2-[2-[Aethyl[3,4-(methylendioxy)phenäthyl]-amino]äthyl]-6-fluor-1,2,3,4-tetrahydro-1-isopropyl-2-naphthalinol als ein leicht braunes Oel, welches direkt in die nächste Stufe eingesetzt wird;
- aus [1R,2R]-(-)-2-[6-Fluor-1,2,3,4-tetrahydro-2-hydroxy-1-isopropyl-2-naphthyl]äthyl-p-toluolsulfonat und 3,4-(Methylendioxy)-N-benzyl-β-phenäthylamin das [1R,2R]-(-)-2-[2-[Benzyl[3,4-(methylendioxy)phenäthyl]-amino]äthyl]-6-fluor-1,2,3,4-tetrahydro-1-isopropyl-2-naphthalinol als ein leicht gelbes Oel, welches direkt in die nächste Stufe eingesetzt wird;
- aus [1R,2R]-(-)-2-[6-Fluor-1,2,3,4-tetrahydro-2-hydroxy-1-isopropyl-2-naphthyl]äthyl-p-toluolsulfonat und 3,4-(Aethylendioxy)-N-methyl-β-phenäthylamin das [1R,2R]-(-)-6-Fluor-1,2,3,4-tetrahydro-1-isopropyl-2-[2-[methyl[3,4-(äthylendioxy)phenäthyl]amino]äthyl]-2-naphthalinol als eine leicht gelbe Masse, welche direkt in die nächste Stufe eingesetzt wird;
- aus [1R,2R]-(-)-2-[6-Fluor-1,2,3,4-tetrahydro-2-hydroxy-1-isopropyl-2-naphthyl]äthyl-p-toluolsulfonat und N-Methyl-3,4-dimethoxy-γ-phenylpropylamin das [1R,2R]-(-)-2-[2-[[3-(3,4-Dimethoxyphenyl)propyl]-methylamino]äthyl]-6-fluor-1,2,3,4-tetrahydro-1-isopropyl-2-naphthalinol als ein leicht gelbes Oel, welches direkt in die nächste Stufe eingesetzt wird und
- aus [1R,2R]-(-)-2-[6-Fluor-1,2,3,4-tetrahydro-2-hydroxy-1-isopropyl-2-naphthyl]äthyl-p-toluolsulfonat und N-Methyl-3,4-dimethoxy-δ-phenylbutylamin das [1R,2R]-(-)-2-[2-[[4-(3,4-Dimethoxyphenyl)butyl]methylamino]-äthyl]-6-fluor-1,2,3,4-tetrahydro-1-isopropyl-2-naphthalinol als eine leicht gelbe Masse, welche direkt in die nächste Stufe eingesetzt wird.

## Beispiel 2

In analoger Weise wie in Beispiel 1 beschrieben wurden die folgenden Verbindungen hergestellt:

- Aus [1R,2R]-(-)-6-Fluor-1,2,3,4-tetrahydro-1-isopropyl-2-[2-[methyl[3,4-(methylendioxy)phenäthyl]amino]-äthyl]-2-naphthalinol und Acetylchlorid das [1R,2R]-(-)-6-Fluor-1,2,3,4-tetrahydro-1-isopropyl-2-[2-[methyl-[3,4-(methylendioxy)phenäthyl]amino]äthyl]-2-naphthylacetat-hydrochlorid als ein leicht gelber Festkörper, $[\alpha]_D^{20}$ = -36,9° (c=1, Methanol)];
- aus [1R,2R]-(-)-6-Fluor-1,2,3,4-tetrahydro-1-isopropyl-2-[2-[methyl[3,4-(methylendioxy)phenäthyl]amino]-äthyl]-2-naphthalinol und Propionylchlorid das [1R,2R]-(-)-6-Fluor-1,2,3,4-tetrahydro-1-isopropyl-2-[2-[methyl[3,4-(methylendioxy)phenäthyl]amino]äthyl]-2-naphthylpropionat-hydrochlorid als eine leicht braune Masse, $[\alpha]_D^{20}$ = -32.9° (c=1, Methanol);
- aus [1R,2R]-(-)-6-Fluor-1,2,3,4-tetrahydro-1-isopropyl-2-[2-[methyl[3,4-(methylendioxy)phenäthyl]amino]-äthyl]-2-naphthalinol und Methylthioacetylchlorid das [1R,2R]-(-)-6-Fluor-1,2,3,4-tetrahydro-1-isopropyl-2-[2-[methyl[3,4-(methylendioxy)phenäthyl]amino]äthyl]-2-naphthyl(methylthio)acetat-hydrochlorid als eine leicht braune Masse, $[\alpha]_D^{20}$ = -27,5° (c=1, Methanol);
- aus [1R,2R]-(-)-6-Fluor-1,2,3,4-tetrahydro-1-isopropyl2-[2-[methyl[3,4-(methylendioxy)phenäthyl]amino]-äthyl]-2-naphthalinol und 3-Furoylchlorid das [1R,2R]-(-)-6-Fluor-1,2,3,4-tetrahydro-1-isopropyl-2-[2-[methyl-[3,4-methylendioxy]phenäthyl]amino]äthyl]-2-naphthyl-3-furancarboxylathydrochlorid als eine leicht gelbe Masse, $[\alpha]_D^{20}$ = -39,3° (c=1, Methanol);
- aus [1R,2R]-(-)-2-[2-[(3,4-Dimethoxyphenäthyl)methylamino]äthyl]-6-fluor-1,2,3,4-tetrahydro-1-isopropyl-2-naphthalinol und Methoxyacetylchlorid das [1R,2R]-(-)-2-[2-[(3,4-Dimethoxyphenäthyl)methylamino]äthyl]-6-fluor-1,2,3,4-tetrahydro-1-isopropyl-2-naphthylmethoxyacetat-hydrochlorid, Smp. 174-175° = $[\alpha]_D^{20}$ , -35° (c=1. Methanol);
- aus [1R,2R]-(-)-2-[2-[(3,4-Dimethoxyphenäthyl)methylamino]äthyl]-6-fluor-1,2,3,4-tetrahydro-1-isopropyl-2-naphthalinol und Acetylchlorid das [1R,2R]-(-)-2-[2-[(3,4-Dimethoxyphenäthyl)methylamino]äthyl]-6-fluor-1,2,3,4-tetrahydro-1-isopropyl-2-naphthylacetat-hydrochlorid als eine leicht gelbe Masse, $[\alpha]_D^{20}$ = -41° (c=1, Methanol);
- aus [1R,2R]-(-)-2-[2-[(3,4-Dimethoxyphenäthyl)methylamino]äthyl]-6-fluor-1,2,3,4-tetrahydro-1-isopropyl-2-naphthalinol und n-Butyrylchlorid das [1R,2R]-(-)-2-[2-[(3,4-Dimethoxyphenäthyl)methylamino]äthyl]-6-fluor-1,2,3,4-tetrahydro-1-isopropyl-2-naphthylvalerat-hydrochlorid als eine leicht braune Masse, $[\alpha]_D^{20}$ = -31,1° (c=1, Methanol);
- aus [1R,2R]-(-)-6-Fluor-1,2,3,4-tetrahydro-1-isopropyl-2-[2-[(phenäthyl)methylamino]äthyl]-2-naphthalinol und Methoxyacetylchlorid das [1R,2R]-(-)-6-Fluor-1,2,3,4-tetrahydro-1-isopropyl-2-[2-(methylphenäthylamino)äthyl]-2-naphthylmethoxyacetat-hydrochlorid als farblose Kristalle, Smp. 185-190°

(aus Essigester), $[\alpha]_D^{20}$ = -37,8° (c = 1, Methanol);

- aus [1R,2R]-(-)-6-Fluor-1,2,3,4-tetrahydro-1-isopropyl-2[2-[(pmethoxyphenäthyl)methylamino]äthyl]-2-naphthalinol und Methoxyacetylchlorid das [1R,2R]-(-)-6-Fluor-1,2,3,4-tetrahydro-2-[2-[(p-methoxyphenäthyl)-methylamino]äthyl]-1-isopropyl-2-naphthylmethoxyacetat-hydrochlorid als eine leicht gelbliche Masse, $[\alpha]_D^{20}$ = -35,1° (c = 1, Methanol);
- aus [1R,2R]-(-)-2-[2-[Aethyl[3,4-(methylendioxy)phenäthyl]amino]äthyl]-6-fluor-1,2,3,4-tetrahydro-1-isopropyl-2-naphthalinol und Methoxyacetylchlorid das [1R,2R]-(-)-2-[2-[Aethyl[3,4-(methylendioxy)-phenäthyl]amino]äthyl]-6-fluor-1,2,3,4-tetrahydro-1-isopropyl-2-naphthylmethoxyacetathydrochlorid als eine leicht braune Masse, $[\alpha]_D^{20}$ = -22,0° (c = 1, Methanol);
- aus [1R,2R]-(-)-2-[2-[Benzyl[3,4-(methylendioxy)phenäthyl]amino]äthyl]-6-fluor-1,2,3,4-tetrahydro-1-isopropyl-2-naphthalinol und Methoxyacetylchlorid das [1R,2R]-(-)-2- [2-[Benzyl[3,4-(methylendioxy)-phenäthyl]amino]äthyl]-6-fluor-1,2,3,4-tetrahydro-1-isopropyl-2-naphthylmethoxyacetat-hydrochlorid als eine leicht gelbe Masse, $[\alpha]_D^{20}$ = -32,4° (c = 1, Methanol);
- aus [1R,2R]-(-)-6-Fluor-1,2,3,4-tetrahydro-1-isopropyl-2-[2-[methyl[3,4-(äthylendioxy)phenäthyl]amino]-äthyl]-2-naphthalinol und Methoxyacetylchlorid das [1R,2R]-(-)-6-Fluor-1,2,3,4-tetrahydro-1-isopropyl-2-[2-[methyl[3,4-(äthylendioxy)phenäthyl]amino]äthyl]-2-naphthylmethoxyacetat-hydrochlorid als eine leicht braune Masse, $[\alpha]_D^{20}$ = -30,7° (c = 1, Methanol);
- aus [1R,2R]-(-)-2-[2-[[3-(3,4-Dimethoxyphenyl)propyl]methylamino]äthyl]-6-fluor-1,2,3,4-tetrahydro-1-isopropyl-2-naphthalinol und Methoxyacetylchlorid das [1R,2R]-(-)-2-[2-[[3-(3,4-Dimethoxyphenyl)propyl]-methylamino]äthyl]-6-fluor-1,2,3,4-tetrahydro-1-isopropyl-2-naphthylmethoxyacetathydrochlorid als ein leicht gelber Schaum, $[\alpha]_D^{20}$ = -30,5° (c = 1, Methanol) und
- aus [1R,2R]-(-)-2-[2-[[4-(3,4-Dimethoxyphenyl)butyl]methylamino]äthyl]-6-fluor-1,2,3,4-tetrahydro-1-isopropyl-2-naphthalinol und Methoxyacetylchlorid das [1R,2R]-(-)-2-[2-[[4-(3,4-Dimethoxyphenyl)butyl]-methylamino]äthyl]-6-fluor-1,2,3,4-tetrahydro-1-isopropyl-2-naphthylmethoxyacetat-hydrochlorid als ein leicht brauner Schaum, $[\alpha]_D^{20}$ = -27,5° (c = 1, Methanol).

## Beispiel 3

In analoger Weise wie in Beispiel 1 beschrieben wurde aus [1R,2R]-(-)-2-[6-Fluor-1,2,3,4-tetrahydro-2-hydroxy-1-isopropyl-2-naphthyl]äthyl-p-toluolsulfonat und N-Methyl-(2,5-difluorhomoveratrylamin) das entsprechende Naphthalinol als dickflüssiges Oel erhalten, welches direkt mit Methoxyacetylchlorid zu [1R,2R]-(-)-[2-[2-(2,5-Difluor -3,4-di methoxyphenäthyl)methylamino]äthyl]-6-fluor -1,2,3,4-tetrahydro-1-isopropyl -2-naphthylmethoxyacetat-hydrochlorid in Form eines leicht braunen Schaums umgesetzt wurde, $[\alpha]_D^{20}$ = -31,3° (c = 1, Methanol).

## Beispiel 4

In analoger Weise wie in Beispiel 1 beschrieben wurde aus [1R,2R]-(-)-2-[6-Fluor-1,2,3,4-tetrahydro-2-hydroxy-1-isopropyl-2-naphthyl]äthyl-p-toluolsulfonat und N-Methyl-(2-fluorhomoveratrylamin) das entsprechende Naphthalinol als dickflüssiges Oel erhalten, welches direkt mit Methoxyacetylchlorid zu [1R,2R]-(-)-2-[2-[(2-Fluor -3,4-dimethoxyphenäthyl)methylamino]äthyl]-6-fluor-1,2,3,4-tetrahydro-1-isopropyl -2-naphthylmethoxyacetat-hydrochlorid in Form einer leicht braunen Masse umgesetzt wurde, $[\alpha]_D^{20}$ = -31,8° (c = 1, Methanol).

## Beispiel A

| Tabletten | | |
|---|---|---|
| | Zusammensetzung : | |
| 1) | [1R,2R]-(-)-6-Fluor-1,2,3,4-tetrahydro-1-isopropyl-2-[2-[methyl[3,4-(methylendioxy)phenäthyl]amino]äthyl]-2-naphthylmethoxyacetat-hydrochlorid | 75 mg |
| 2) | Milchzucker pulv. | 135 mg |
| 3) | Maisstärke weiss | 55 mg |
| 4) | Povidone K 30 | 15 mg |
| 5) | Maisstärke weiss | 15 mg |
| 6) | Talk | 3 mg |
| 7) | Magnesiumstearat | 2 mg |
| | Tablettengewicht | 300 mg |

Herstellungverfahren:

1-3 werden intensiv gemischt. Die Mischung wird danach mit einer wässrigen Lösung von 4 befeuchtet und geknetet, und die resultierende Masse granuliert, getrocknet und gesiebt. Das Granulat wird mit 5-7 gemischt und zu Tabletten geeigneter Grösse verpresst.

## Beispiel B

| Tabletten | | | |
|---|---|---|---|
| | Zusammensetzung : | | |
| 1) | [1R,2R]-(-)-6-Fluor-1,2,3,4-tetrahydro-1-isopropyl-2-[2-[methyl[3,4-(methylendioxy)phenäthyl]amino]äthyl]-2-naphthylmethoxyacetat-hydrochlorid | 75 mg | 60 mg |
| 2) | Milchzucker pulv. | 100 mg | 100 mg |
| 3) | Maisstärke | 60 mg | 60 mg |
| 4) | Povidone K 30 | 5 mg | 5 mg |
| 5) | Maisstärke | 15 mg | 15 mg |
| 6) | Natriumcarboxymethylstärke | 5 mg | 5 mg |
| 7) | Talk | 3 mg | 3 mg |
| 8) | Magnesiumstearat | 2 mg | 2 mg |
| | Tablettengewicht | 265 mg | 250 mg |

Herstellungverfahren:

1-3 werden intensiv gemischt. Die Mischung wird danach mit einer wässrigen Lösung von 4 befeuchtet und geknetet, und die resultierende Masse granuliert, getrocknet und gesiebt. Das Granulat wird mit 5-7 gemischt und zu Tabletten geeigneter Grösse verpresst.

Beispiel C

| | | | |
|---|---|---|---|
| Tabletten | | | |
| | Zusammensetzung : | | |
| 1) | [1R,2R]-(-)-6-Fluor-1,2,3,4-tetrahydro-1-isopropyl-2-[2-[methyl[3,4-(methylendioxy)phenäthyl]amino]äthyl]-2-naphthylmethoxyacetat-hydrochlorid | 75 mg | 90 mg |
| 2) | Milchzucker pulv. | 46 mg | 46 mg |
| 3) | Cellulose mikrokristallin | 60 mg | 60 mg |
| 4) | Povidone K 30 | 10 mg | 10 mg |
| 5) | Natriumcarboxymethylstärke | 4 mg | 4 mg |
| 6) | Talk | 3 mg | 3 mg |
| 7) | Magnesiumstearat | 2 mg | 2 mg |
| | Tablettengewicht | 200 mg | 215 mg |

Herstellungverfahren:

1-3 werden intensiv gemischt. Die Mischung wird danach mit einer wässrigen Lösung von 4 befeuchtet und geknetet, und die resultierende Masse granuliert, getrocknet und gesiebt. Das Granulat wird mit 5-7 gemischt und zu Tabletten geeigneter Grösse verpresst.

Beispiel D

| | | |
|---|---|---|
| Kapseln | | |
| | Zusammensetzung : | |
| 1) | [1R,2R]-(-)-6-Fluor-1,2,3,4-tetrahydro-1-isopropyl-2-[2-[methyl[3,4-(methylendioxy)phenäthyl]amino]äthyl]-2-naphthylmethoxyacetat-hydrochlorid | 75 mg |
| 2) | Milchzucker krist. | 100 mg |
| 3) | Maisstärke weiss | 20 mg |
| 4) | Talk | 9 mg |
| 5) | Magnesiumstearat | 1 mg |
| | Kapselfüllgewicht | 205 mg |

Herstellungverfahren:

Der Wirkstoff wird mit dem Milchzucker intensiv gemischt. Dieser Mischung wird danach die Maisstärke, der Talk und das Magnesiumstearat zugemischt, und das Gemisch in Kapseln geeigneter Grösse abgefüllt.

Beispiel E

| | Kapseln | |
|---|---|---|
| | Zusammensetzung : | |
| 1) | [1R,2R]-(-)-6-Fluor-1,2,3,4-tetrahydro-1-isopropyl-2-[2-[methyl[3,4-(methylendioxy)phenäthyl]amino]äthyl]-2-naphthylmethoxyacetat-hydrochlorid | 75 mg |
| 2) | Cellulose mikrokristallin | 100 mg |
| 3) | Natriumcarboxymethylstärke | 5 mg |
| 4) | Talk | 9 mg |
| 5) | Magnesiumstearat | 1 mg |
| | Kapselfüllgewicht | 190 mg |

Herstellungverfahren:

Der Wirkstoff wird mit der Cellulose intensiv gemischt. Dieser Mischung wird danach die Natriumcarboxymethylstärke, der Talk und das Magnesiumstearat zugemischt, und das Gemisch in Kapseln geeigneter Grösse abgefüllt.

## Beispiel F

| Injektionslösung | |
|---|---|
| [1R,2R]-(-)-6-Fluor-1,2,3,4-tetrahydro-1-isopropyl-2-[2-[methyl[3,4-(methylendioxy)phenäthyl]amino]äthyl]-2-naphthylmethoxyacetat-hydrochlorid | 8 mg |
| Natriumchlorid krist. rein | 8,5 mg |
| Wasser zu Injektionszwecken | ad 1 ml |

Beispiel G

Wenn man nach den in den Beispielen A-F beschriebenen Verfahren arbeitet, können aus den folgenden, ebenfalls bevorzugten Verbindungen und ihren pharmazeutisch verwendbaren Salzen Tabletten, Kapseln und Injektionspräparate hergestellt werden:
[1R,2R]-(-)-2-[2-[(3,4-Dimethoxyphenäthyl)methylamino]äthyl]-6-fluor-1,2,3,4-tetrahydro-1-isopropyl-2-naphthylmethoxyacetat-hydrochlorid,
[1R,2R]-(-)-6-Fluor-1,2,3,4-tetrahydro-2-[2-[(p-methoxyphenäthyl)methylamino]äthyl]-1-isopropyl-2-naphthylmethoxyacetat-hydrochlorid,
[1R,2R]-(-)-6-Fluor-1,2,3,4-tetrahydro-1-isopropyl-2-[2-[methyl[3,4-(äthylendioxy)phenäthyl]amino]äthyl]-2-naphthylmethoxyacetat-hydrochlorid,
[1R,2R]-(-)-2-[2-[[3-(3,4-Dimethoxyphenyl)propyl]methylamino]äthyl]-6-fluor-1,2,3,4-tetrahydro-1-isopropyl-2-naphthyl methoxyacetat-hydrochlorid und
[1R,2R]-(-)-2-[2-[[4-(3,4-Dimethoxyphenyl)butyl]methylamino]äthyl]-6-fluor-1,2,3,4-tetrahydro-1-isopropyl-2-naphthylmethoxyacetat-hydrochlorid.

**Ansprüche**

1. Tetrahydronaphthalinderivate der allgemeinen Formel

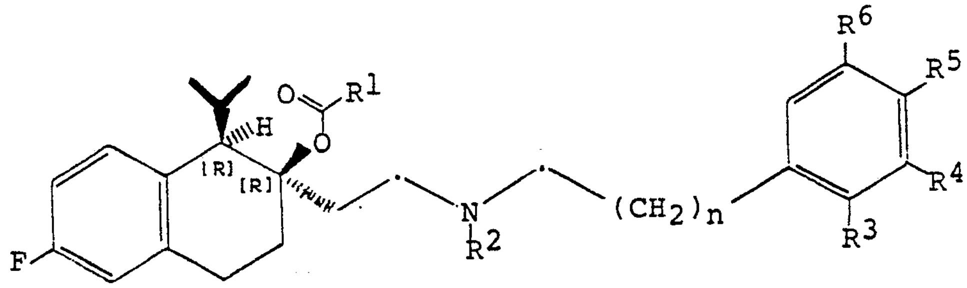

I

worin $R^1$ nieder-Alkyl, nieder-Alkoxy-nieder-alkyl, nieder-Alkylthio-nieder-alkyl oder Furyl, $R^2$ nieder-Alkyl, Phenyl-nieder-alkyl oder Cyclohexyl-nieder-alkyl, $R^3$ und $R^6$ je Wasserstoff oder Fluor, $R^4$ und $R^5$ je Wasserstoff oder nieder-Alkoxy oder zusammen Methylendioxy, Aethylendioxy oder Aethylenoxy und n die Zahl 1, 2 oder 3 bedeuten,
sowie pharmazeutisch verwendbare Säureadditionssalze davon.

2. Verbindungen gemäss Anspruch 1, worin $R^1$ nieder-Alkoxy-nieder-alkyl oder Furyl, bevorzugt Methoxymethyl oder 2-Furyl, bedeutet.

3. Verbindungen gemäss Anspruch 1 oder 2, worin n die Zahl 1 bedeutet.

4. Verbindungen gemäss einem der Ansprüche 1-3, worin $R^2$ nieder-Alkyl, bevorzugt Methyl, bedeutet.

5. Verbindungen gemäss einem der Ansprüche 1-4, worin $R^3$ und $R^6$ Wasserstoff bedeuten.

6. Verbindungen gemäss einem der Ansprüche 1-5, worin $R^4$ und $R^5$ je Wasserstoff oder nieder-Alkoxy, bevorzugt Methoxy, oder zusammen Methylendioxy bedeuten.

7. Verbindungen gemäss einem der Ansprüche 1-6, worin $R^1$ Methoxymethyl oder 2-Furyl, $R^2$ Methyl, $R^3$ und $R^6$ Wasserstoff, $R^4$ und $R^5$ je Wasserstoff, Methoxy oder zusammen Methylendioxy und n die Zahl 1 bedeuten.

8. [1R,2R]-(-)-6-Fluor-1,2,3,4-tetrahydro-1-isopropyl-2-[2-[methyl-[3,4-(methylendioxy)phenäthylamino]-äthyl]-2-naphthylmethoxyacetat.

9. [1R,2R]-(-)-2-[2-[(3,4-Dimethoxyphenäthyl)methylamino]äthyl]-6-fluor-1,2,3,4-tetrahydro-1-isopropyl-2-naphthylmethoxyacetat.

10. [1R,2R]-(-)-6-Fluor-1,2,3,4-tetrahydro-2-[2-[(p-methoxyphenäthyl)methylamino]äthyl]-1-isopropyl-2-naphthylmethoxyacetat.

11. [1R,2R]-(-)-6-Fluor-1,2,3,4-tetrahydro-1-isopropyl-2-[2-[methyl[3,4-(äthylendioxy)phenäthyl]-methylamino]äthyl]-2-naphthylmethoxyacetat.

12. [1R,2R]-(-)-2-[2-[[3-(3,4-Dimethoxyphenyl)propyl]methylamino]äthyl]-6-fluor-1,2,3,4-tetrahydro-1-isopropyl-2-naphthylmethoxyacetat.

13. [1R,2R]-(-)-2-[2-[[4-(3,4-Dimethoxyphenyl)butyl]methylamino]äthyl]-6-fluor-1,2,3,4-tetrahydro-1-isopropyl-2-naphthylmethoxyacetat.

14. Verbindungen der allgemeinen Formel

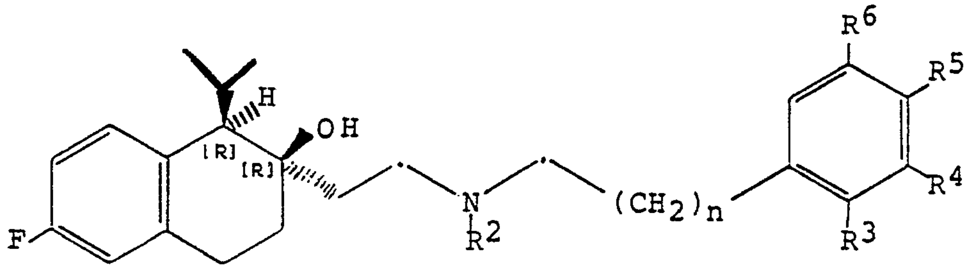

II

worin $R^2$ nieder-Alkyl, Phenyl-nieder-alkyl oder Cyclohexoyl-nieder-alkyl, $R^3$ und $R^6$ je Wasserstoff oder Fluor, $R^4$ und $R^5$ je Wasserstoff oder nieder-Alkoxy oder zusammen Methylendioxy, Aethylendioxy oder Aethylenoxy und n die Zahl 1, 2 oder 3 bedeuten.

15. Verbindungen der allgemeinen Formel

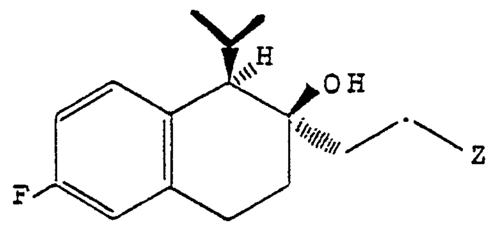

worin Z Hydroxy oder eine Abgangsgruppe bedeutet.

16. Verbindungen der allgemeinen Formel

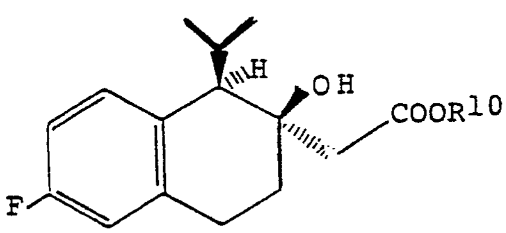

VI

worin $R^{10}$ Wasserstoff oder nieder-Alkyl bedeutet.

17. Tetrahydronaphthalinderivate gemäss einem der Ansprüche 1-13 zur Anwendung als therapeutische Wirkstoffe.

18. Tetrahydronaphthalinderivate gemäss einem der Ansprüche 1-13 zur Anwendung bei der Bekämpfung bzw. Verhütung cerebraler Insuffizienz bzw. bei der Verbesserung cognitiver Funktionen sowie zur Verminderung der Mehrfach-Resistenz gegenüber Cytostatika bei der Behandlung von Tumoren bzw. Chloroquinresistenz bei der Behandlung von Malaria.

19. Verfahren zur Herstellung einer Verbindung gemäss einem der Ansprüche 1-13, dadurch gekennzeichnet, dass man eine Verbindung der allgemeinen Formel

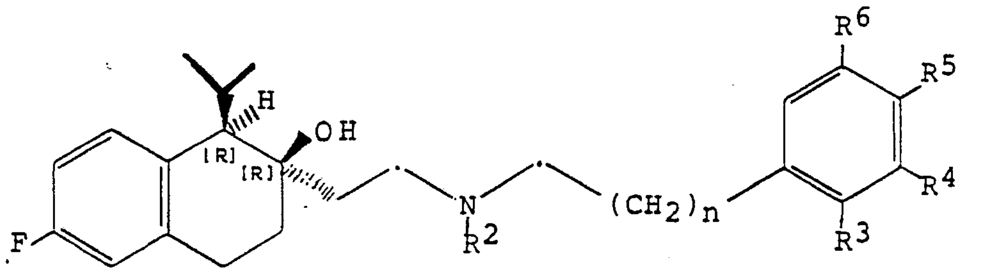

II

worin $R^2$, $R^3$, $R^4$, $R^5$, $R^6$ und n die in Anspruch 1 angegebene Bedeutung besitzen, mit einem eine nieder-Alkylcarbonyl-, nieder-Alkoxy-nieder-alkylcarbonyl-, nieder-Alkylthio-nieder-alkylcarbonyl- oder Furylcarbonyl-Gruppe abgebenden Acylierungsmittel umsetzt, und erwünschtenfalls, eine erhaltene Verbindung in ein pharmazeutisch verwendbares Säureadditionssalz überführt.

20. Arzneimittel, enthaltend ein Tetrahydronaphthalinderivat gemäss einem der Ansprüche 1-13 und ein therapeutisch inertes Excipiens.

21. Mittel zur Bekämpfung bzw. Verhütung cerebraler Insuffizienz bzw. zur Verbesserung cognitiver Funktionen sowie zur Verminderung der Mehrfach-Resistenz gegenüber Cytostatika bei der Behandlung von Tumoren bzw. Chloroquinresistenz bei der Behandlung von Malaria, enthaltend ein Tetrahydronaphthalinderivat gemäss einem der Ansprüche 1-13 und ein therapeutisch inertes Excipiens.

22. Verwendung eines Tetrahydronaphthalinderivates gemäss einem der Ansprüche 1-13 bei der Bekämpfung bzw. Verhütung von Krankheiten.

23. Verwendung eines Tetrahydronaphthalinderivates gemäss einem der Ansprüche 1-13 bei der Bekämpfung bzw. Verhütung der cerebralen Insuffizienz bzw. bei der Verbesserung cognitiver Funktionen sowie zur Verminderung der Mehrfach-Resistenz gegenüber Cytostatika bei der Behandlung von Tumoren bzw. Chloroquinresistenz bei der Behandlung von Malaria.

24. Verwendung eines Tetrahydronaphthalinderivates gemäss einem der Ansprüche 1-13 zur Herstellung von der cerebralen Insuffizienz entgegenwirkenden bzw. cognitive Funktionen verbessernden Mitteln sowie von Mitteln zur Verminderung der Mehrfach-Resistenz gegenüber Cytostatika bei der Behandlung von Tumoren bzw. Chloroquinresistenz bei der Behandlung von Malaria.

Patentansprüche für folgenden Vertragsstaat: ES

1. Verfahren zur Herstellung von Tetrahydronaphthalinderivaten der allgemeinen Formel

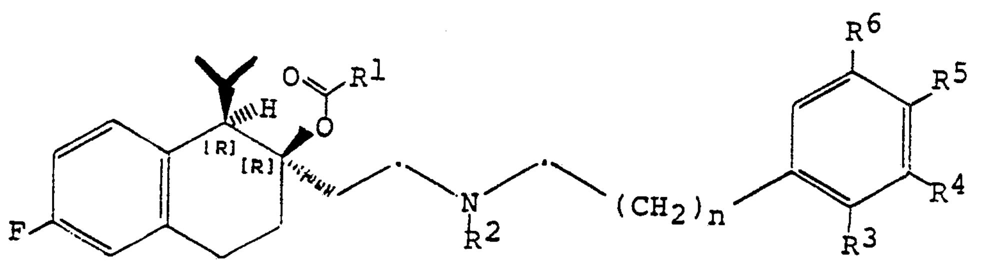

I

worin $R^1$ nieder-Alkyl, nieder-Alkoxy-nieder-alkyl, nieder-Alkylthio-nieder-alkyl oder Furyl, $R^2$ nieder-Alkyl, Phenyl-nieder-alkyl oder Cyclohexyl-nieder-alkyl, $R^3$ und $R^6$ je Wasserstoff oder Fluor, $R^4$ und $R^5$ je Wasserstoff oder nieder-Alkoxy oder zusammen Methylendioxy, Aethylendioxy oder Aethylenoxy und n die Zahl 1, 2 oder 3 bedeuten,
sowie pharmazeutisch verwendbaren Säureadditionssalzen davon, dadurch gekennzeichnet, dass man eine Verbindung der allgemeinen Formel

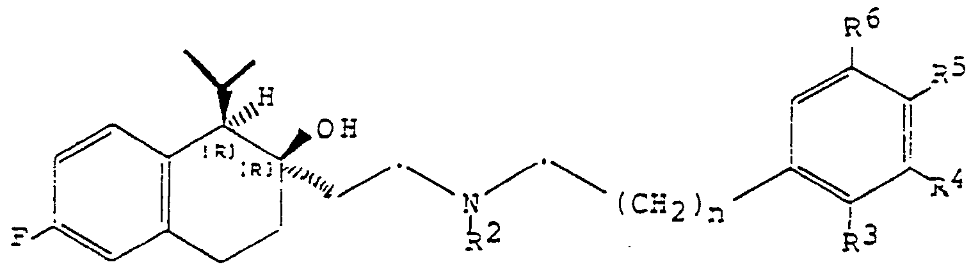

II

worin $R^2$, $R^3$, $R^4$, $R^5$, $R^6$ und n die oben angegebene Bedeutung besitzen, mit einem eine nieder-Alkylcarbonyl-, nieder-Alkoxy-nieder-alkylcarbonyl-, nieder-Alkylthio-nieder-alkylcarbonyl- oder Furylcarbonyl-Gruppe abgebenden Acylierungsmittel umsetzt, und erwünschtenfalls, eine erhaltene Verbindung in ein pharmazeutisch verwendbares Säureadditionssalz überführt.

2. Verfahren gemäss Anspruch 1, worin $R^1$ nieder-Alkoxy-nieder-alkyl oder Furyl, bevorzugt Methoxymethyl oder 2-Furyl, bedeutet.

3. Verfahren gemäss Anspruch 1 oder 2, worin n die Zahl 1 bedeutet.

4. Verfahren gemäss einem der Ansprüche 1-3, worin $R^2$ nieder-Alkyl, bevorzugt Methyl, bedeutet.

5. Verfahren gemäss einem der Ansprüche 1-4, worin $R^3$ und $R^6$ Wasserstoff bedeuten.

6. Verfahren gemäss einem der Ansprüche 1-5, worin $R^4$ und $R^5$ je Wasserstoff oder nieder-Alkoxy, bevorzugt Methoxy, oder zusammen Methylendioxy bedeuten.

7. Verfahren gemäss einem der Ansprüche 1-6, worin $R^1$ Methoxymethyl oder 2-Furyl, $R^2$ Methyl, $R^3$ und $R^6$ Wasserstoff, $R^4$ und $R^5$ je Wasserstoff, Methoxy oder zusammen Methylendioxy und n die Zahl 1 bedeuten.

8. Verfahren gemäss Anspruch 1, dadurch gekennzeichnet, dass man [1R,2R]-(-)-6-Fluor-1,2,3,4-tetrahydro-1-isopropyl-2-[2-[methyl-[3,4-(methylendioxy)phenäthylamino]äthyl]-2-naphthylmethoxyacetat herstellt.

9. Verfahren gemäss Anspruch 1, dadurch gekennzeichnet, dass man [1R,2R]-(-)-2-[2-[(3,4-Dimethoxyphenäthyl)methylamino]äthyl]-6-fluor-1,2,3,4-tetrahydro-1-isopropyl-2-naphthylmethoxyacetat herstellt.

10. Verfahren gemäss Anspruch 1, dadurch gekennzeichnet, dass man [1R,2R]-(-)-6-Fluor-1,2,3,4-tetrahydro-2-[2-[(p-methoxyphenäthyl)methylamino]äthyl]-1-isopropyl-2-naphthylmethoxyacetat herstellt.

11. Verfahren gemäss Anspruch 1, dadurch gekennzeichnet, dass man [1R,2R]-(-)-6-Fluor-1,2,3,4-tetrahydro-1-isopropyl-2-[2-[methyl[3,4-(äthylendioxy)phenäthyl]methylamino]äthyl]-2-naphthylmethoxyacetat herstellt.

12. Verfahren gemäss Anspruch 1, dadurch gekennzeichnet, dass man [1R,2R]-(-)-2-[2-[[3-(3,4-Dimethoxyphenyl)propyl]methylamino]äthyl]-6-fluor-1,2,3,4-tetrahydro-1-isopropyl-2-naphthyl-methoxyacetat herstellt.

13. Verfahren gemäss Anspruch 1, dadurch gekennzeichnet, dass man [1R,2R]-(-)-2-[2-[[4-(3,4-Dimethoxyphenyl)butyl]methylamino]äthyl]-6-fluor-1,2,3,4-tetrahydro-1-isopropyl-2-naphthylmethoxyacetat herstellt.

14. Verfahren zur Herstellung von Arzneimitteln, inbesondere zur Verwendung bei der Bekämpfung bzw. Verhütung von cerebraler Insuffizienz bzw. bei der Verbesserung cognitiver Funktionen sowie zur Verminderung der Mehrfach-Resistenz gegenüber Cytostatika bei der Behandlung von Tumoren bzw. Chloroquinresistenz bei der Behandlung von Malaria dadurch gekennzeichnet, dass man ein Tetrahydronaphthalinderivat der in Anspruch 1 angegebenen Formel I oder ein pharmazeutisch verwendbares Säureadditionssalz davon in eine galenische Darreichungsform bringt.

15. Verwendung eines Tetrahydronaphthalinderivates der in Anspruch 1 angegebenen Formel I oder eines pharmazeutisch verwendbaren Säureadditionssalzes davon zur Herstellung von der cerebralen Insuffizienz entgegenwirkenden bzw. cognitive Funktionen verbessernden Mitteln sowie von Mitteln zur Verminderung der Mehrfach-Resistenz gegenüber Cytostatika bei der Behandlung von Tumoren bzw. Chloroquinresistenz bei der Behandlung von Malaria.

Patentansprüche für folgenden Vertragsstaat: GR

1. Verfahren zur Herstellung von Tetrahydronaphthalinderivaten der allgemeinen Formel

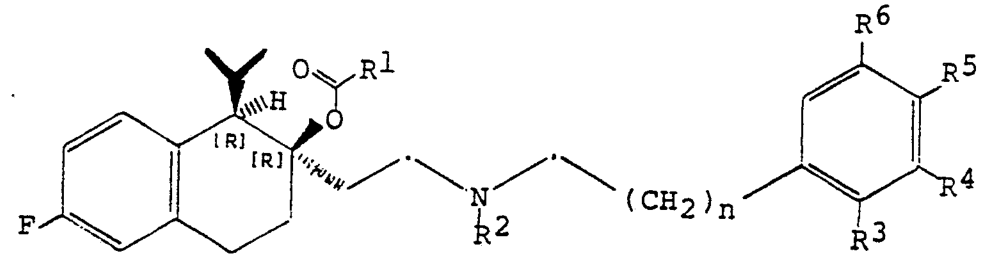

I

worin $R^1$ nieder-Alkyl, nieder-Alkoxy-nieder-alkyl, nieder-Alkylthio-nieder-alkyl oder Furyl, $R^2$ nieder-Alkyl, Phenyl-nieder-alkyl oder Cyclohexyl-nieder-alkyl, $R^3$ und $R^6$ je Wasserstoff oder Fluor, $R^4$ und $R^5$ je Wasserstoff oder nieder-Alkoxy oder zusammen Methylendioxy, Aethylendioxy oder Aethylenoxy und n die Zahl 1, 2 oder 3 bedeuten,
sowie pharmazeutisch verwendbaren Säureadditionssalzen davon, dadurch gekennzeichnet, dass man eine Verbindung der allgemeinen Formel

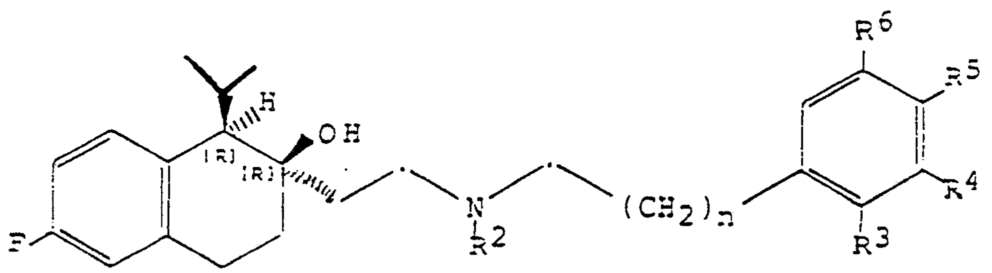

II

worin $R^2$, $R^3$, $R^4$, $R^5$, $R^6$ und n die oben angegebene Bedeutung besitzen,
mit einem eine nieder-Alkylcarbonyl-, nieder-Alkoxy-nieder-alkylcarbonyl-, nieder-Alkylthio-nieder-alkylcarbonyl- oder Furylcarbonyl-Gruppe abgebenden Acylierungsmittel umsetzt, und erwünschtenfalls, eine erhaltene Verbindung in ein pharmazeutisch verwendbares Säureadditionssalz überführt.

2. Verfahren gemäss Anspruch 1, worin $R^1$ nieder-Alkoxy-nieder-alkyl oder Furyl, bevorzugt Methoxymethyl oder 2-Furyl, bedeutet.

3. Verfahren gemäss Anspruch 1 oder 2, worin n die Zahl 1 bedeutet.

4. Verfahren gemäss einem der Ansprüche 1-3, worin $R^2$ nieder-Alkyl, bevorzugt Methyl, bedeutet.

5. Verfahren gemäss einem der Ansprüche 1-4, worin $R^3$ und $R^6$ Wasserstoff bedeuten.

6. Verfahren gemäss einem der Ansprüche 1-5, worin $R^4$ und $R^5$ je Wasserstoff oder nieder-Alkoxy, bevorzugt Methoxy, oder zusammen Methylendioxy bedeuten.

7. Verfahren gemäss einem der Ansprüche 1-6, worin $R^1$ Methoxymethyl oder 2-Furyl, $R^2$ Methyl, $R^3$ und $R^6$ Wasserstoff, $R^4$ und $R^5$ je Wasserstoff, Methoxy oder zusammen Methylendioxy und n die Zahl 1 bedeuten.

8. Verfahren gemäss Anspruch 1, dadurch gekennzeichnet, dass man [1R,2R]-(-)-6-Fluor-1,2,3,4-tetrahydro-1-isopropyl-2-[2-[methyl-[3,4-(methylendioxy)phenäthylamino]äthyl]-2-naphthylmethoxyacetat herstellt.

9. Verfahren gemäss Anspruch 1, dadurch gekennzeichnet, dass man [1R,2R]-(-)2-[2-[(3,4-Dimethoxyphenäthyl)methylamino]äthyl]-6-fluor-1,2,3,4-tetrahydro-1-isopropyl-2-naphthylmethoxyacetat herstellt.

10. Verfahren gemäss Anspruch 1, dadurch gekennzeichnet, dass man [1R,2R]-(-)-6-Fluor-1,2,3,4-tetrahydro-2-[2-[(p-methoxyphenäthyl)methylamino]äthyl]-1-isopropyl-2-naphthylmethoxyacetat herstellt.

11. Verfahren gemäss Anspruch 1, dadurch gekennzeichnet, dass man [1R,2R]-(-)-6-Fluor-1,2,3,4-tetrahydro-1-isopropyl-2-[2-[methyl[3,4-(äthylendioxy)phenäthyl]methylamino]äthyl]-2-naphthylmethoxyacetat herstellt.

12. Verfahren gemäss Anspruch 1, dadurch gekennzeichnet, dass man [1R,2R]-(-)-2-[2-[[3-(3,4-Dimethoxyphenyl)propyl]methylamino]äthyl]-6-fluor-1,2,3,4-tetrahydro-1-isopropyl-2-naphthyl-methoxyacetat herstellt.

13. Verfahren gemäss Anspruch 1, dadurch gekennzeichnet dass man [1R,2R]-(-)-2-[2-[[4-(3,4-Dimethoxyphenyl)butyl]methylamino]äthyl]-6-fluor-1,2,3,4-tetrahydro-1-isopropyl-2-naphthylmethoxyacetat herstellt.

14. Verfahren zur Herstellung von Arzneimitteln, insbesondere zur Verwendung bei der Bekämpfung bzw. Verhütung von cerebraler Insuffizienz bzw. bei der Verbesserung cognitiver Funktionen sowie zur Verminderung der Mehrfach-Resistenz gegenüber Cytostatika bei der Behandlung von Tumoren bzw. Chloroquinresistenz bei der Behandlung von Malaria, dadurch gekennzeichnet, dass man ein Tetrahydronaphthalinderivat der in Anspruch 1 angegebenen Formel I oder ein pharmazeutisch verwendbares Säureadditionssalz davon in eine galenische Darreichungsform bringt.

15. Verwendung eines Tetrahydronaphthalinderivates der in Anspruch 1 angegebenen Formel I oder eines pharmazeutisch verwendbaren Säureadditionssalzes davon zur Herstellung von der cerebralen Insuffizienz entgegenwirkenden bzw. cognitive Funktionen verbessernden Mitteln sowie von Mitteln zur Verminderung der Mehrfach-Resistenz gegenüber Cytostatika bei der Behandlung von Tumoren bzw. Chloroquinresistenz bei der Behandlung von Malaria.

16. Verbindungen der allgemeinen Formel

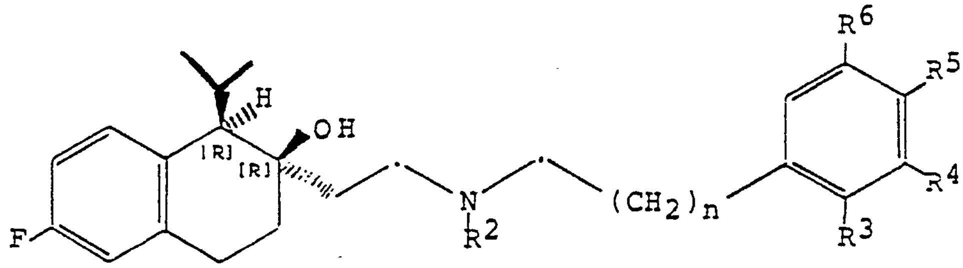

II

worin $R^2$ nieder-Alkyl, Phenyl-nieder-alkyl oder Cyclohexoyl-nieder-alkyl, $R^3$ und $R^6$ je Wasserstoff oder Fluor, $R^4$ und $R^5$ je Wasserstoff oder nieder-Alkoxy oder zusammen Methylendioxy, Aethylenadioxy oder Aethylenoxy und n die Zahl 1, 2 oder 3 bedeuten.

17. Verbindungen der allgemeinen Formel

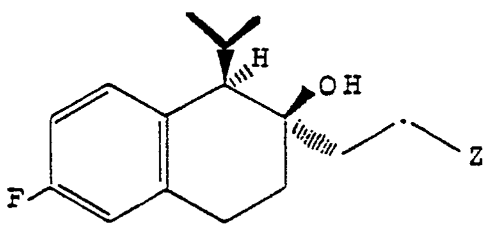

worin Z Hydroxy oder eine Abgangsgruppe bedeutet.

18. Verbindungen der allgemeinen Formel

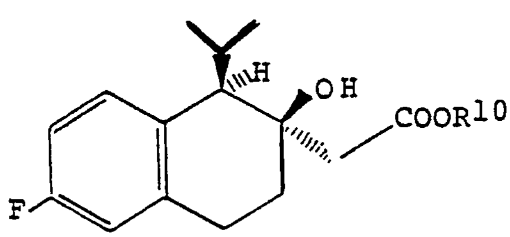

VI

worin $R^{10}$ Wasserstoff oder nieder-Alkyl bedeutet.

Europäisches Patentamt

# EUROPÄISCHER RECHERCHENBERICHT

Nummer der Anmeldung

EP 90 10 4562

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| A<br>D | EP-A-0177960 (HOFFMANN LA ROCHE)<br>* das ganze Dokument *<br>& US-A-4680310 | 1-20 | C07C219/00<br>C07C323/22<br>C07D307/46<br>A61K31/215 |
| A | EP-A-268148 (HOFFMANN LA ROCHE)<br>* das ganze Dokument * | 1-20 | |
| A | EP-A-80721 (SUMITOMO)<br>* Ansprüche 1-42 * | 1, 17, 20, 22 | |
| A,P | CHEMICAL ABSTRACTS, vol. 111, no. 1, 03 Juli 1989<br>Columbus, Ohio, USA<br>"in vitro pharmacologic profile of ro 40-5967,a novel calcium channel blocker with potent vasodilator but weak inotropic action"<br>Seite 40; Spalte 1; ref. no. 442F<br>* Zusammenfassung * | 1 | |
| | | | RECHERCHIERTE SACHGEBIETE (Int. Cl.5 ) |
| | | | C07C<br>A61K<br>C07D |

Der vorliegende Recherchenbericht wurde für alle Patentansprüche erstellt

| Recherchenort | Abschlußdatum der Recherche | Prüfer |
|---|---|---|
| BERLIN | 27 APRIL 1990 | RUFET, J |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument



EPO FORM 1503 03.82 (P0403)